(19) Europäisches Patentamt / European Patent Office / Office européen des brevets

(11) **EP 3 075 375 B1**

(12) **EUROPEAN PATENT SPECIFICATION**

(45) Date of publication and mention
of the grant of the patent:
**15.05.2019 Bulletin 2019/20**

(21) Application number: **14865159.9**

(22) Date of filing: **20.11.2014**

(51) Int Cl.:
*A61K 8/34* (2006.01)  *A61K 8/81* (2006.01)
*A61Q 19/00* (2006.01)

(86) International application number:
**PCT/JP2014/080713**

(87) International publication number:
**WO 2015/080011 (04.06.2015 Gazette 2015/22)**

(54) **SKIN EXTERNAL PREPARATION**

EXTERNES HAUTPRÄPARAT

PRÉPARATION EXTERNE POUR LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.11.2013 JP 2013247210**

(43) Date of publication of application:
**05.10.2016 Bulletin 2016/40**

(73) Proprietor: **Kao Corporation
Chuo-ku
Tokyo 103-8210 (JP)**

(72) Inventors:
• **SUGITA, Jun
Odawara-shi
Kanagawa 250-0002 (JP)**
• **YAKUMARU, Masafumi
Odawara-shi
Kanagawa 250-0002 (JP)**
• **SATOU, Mayumi
Odawara-shi
Kanagawa 250-0002 (JP)**

(74) Representative: **Hoffmann Eitle
Patent- und Rechtsanwälte PartmbB
Arabellastraße 30
81925 München (DE)**

(56) References cited:
WO-A1-2006/061678    JP-A- S 627
JP-A- H0 648 914    JP-A- H11 322 575
JP-A- 2006 188 674    KR-A- 20130 056 979

• 'Sensual Body Lotion' MINTEL GNPD December
2010, XP008183676 Retrieved from the Internet:
<URL:http://www.gnpd.com>
• MINTEL GNPD: 'Exfoliating Soap' December
2007, XP008183873 Retrieved from the Internet:
<URL:http://www.gnpd.com>

EP 3 075 375 B1

**Description**

Field of the Invention

The present invention relates to a skin external preparation.

Background of the Invention

Conventionally, a cosmetic containing a moisturizing agent has been used as one of the measures against rough skin. In cosmetics expected to provide a moisturizing effect, for example, a water-soluble polyol, a saccharide, and a mucopolysaccharide are often formulated. However, in most cosmetics containing these, higher amount of moisturizing agents serves to moisturizing effect, on the other hand it causes sticky feeling. Thus they were not preferable in view of a feeling on use.

For the purpose of cosmetics having moisturizing effect and less sticky feeling, reported are cosmetics containing raffinose (Patent Literature 1) and cosmetics containing a specific polysaccharide or a specific oligosaccharide (Patent Literatures 2 and 3).

If the texture of the skin surface becomes coarse due to rough skin, brightness of the skin decreases, and the skin tone changes from red to yellow, which causes dullness. Accordingly, it is believed that using cosmetics containing moisturizing agent to fix the skin texture and to smoothen the skin surface leads to prevention and improvement of dullness. However, with cosmetics simply containing a moisturizing agent only, prevention and improvement of dullness is still insufficient. If the moisturizing agent is formulated in a large amount, it is further not preferable in view of a feeling on use as well.

Accordingly, cosmetics were developed in which, for example, a blood circulation promoter, a skin whitening agent, and an antioxidant are combined, which are generally considered to be excellent in prevention and improvement of dullness effect (Patent Literature 4).

Patent Literature 1: JP 4351749 B2
Patent Literature 2: JP H10-237105 A
Patent Literature 3: JP H10-25234 A
Patent Literature 4: JP 4683861 B2

JPH0648914 suggest to use pentaerythritol as humectant. WO2006/061678 suggests the use of polyols as skin care actives.

Summary of the Invention

The present invention provides a skin external preparation comprising the following ingredients (A) to (C) :

(A) from 0.1 to 10% by mass of pentaerythritol;
(B) from 0.1 to 25% by mass of one or more kinds selected from the group consisting of glycerin, 1,3-butylene glycol, propanediol, dipropylene glycol, 1,4-butylene glycol, diglycerin, threitol, erythritol, sorbitol and mannitol,
(C) one or two or more of a water-soluble polymer selected from a carboxyvinyl polymer and an acrylic acid-alkylmethacrylate copolymer,

wherein the mass ratio of the content of the ingredient (A) relative to the total content of the ingredient (A) and the ingredient (B) [A/(A + B)] is from 0.03 to 0.8.

In addition, the present invention provides use of a skin external preparation comprising the above-described ingredients (A) to (C) according to claim 5 for improving skin moisturizing performance, elasticity of the skin or dull feeling of the skin.

Furthermore, the present invention provides a method for improving skin moisturizing performance, elasticity of the skin or dull feeling of the skin, characterized by applying a composition comprising the above-described ingredients (A) to (C) according to claim 6 to the skin.

Detailed Description of the Invention

If the concentration of a polyalcohol or a specific saccharide increases in order to obtain sufficient moisturizing effect, problems of undesirable feeling on use such as sticky feeling occur. In addition, combined use of other ingredients than a moisturizing agent is considered to be effective in order to obtain sufficient effect of preventing and improving dullness. However, in this case, problems in stability and safety occur.

Accordingly, the present invention provides a skin external preparation that is free of sticky feeling, and is excellent in a feeling on use such as moisturizing feeling and elasticity, and further also excellent in an effect of preventing and improving dullness of the skin.

The present inventors conducted earnest investigation considering the situations described above, and as a result thereof, found that a skin external preparation comprising (A) pentaerythritol and (B) one or more kinds selected from the group consisting of $C_{3-6}$ polyalcohol and sugar alcohol except pentaerythritol, in combination with component (C) as defined in claim 1 does not generate sticky feeling and is excellent in a feeling on use such as moisturizing feeling and elasticity, and also excellent in an effect of preventing and improving dullness of the skin, and completed the present invention.

The skin external preparation of the present invention has no generation of sticky feeling and is excellent in a feeling on use such as moisturizing feeling and elasticity, and also excellent in an effect of preventing and improving dullness of the skin, also excellent in stability.

Hereinafter, the constitutions of the present invention will be explained in detail.

Pentaerythritol, which is the ingredient (A) of the present invention, is a kind of a sugar alcohol represented by formula $C(CH_2OH)_4$.

The content of the ingredient (A) in the present invention is 0.1% or more by mass, preferably 0.5% or more by mass and more preferably 1% or more by mass, and is 10% or less by mass, preferably 8% or less by mass and more preferably 5% or less by mass on the basis of the total amount of the skin external preparation. The specific range of pentaerythritol content is preferably from 0.5 to 8% by mass and more preferably from 1 to 5% by mass. Within this range, the moisturizing effect of the ingredient (B) can be maintained, and there is no generation of sticky feeling, and a feeling on use excellent in elasticity and plump feeling can be obtained, and further an effect of preventing and improving dullness of the skin can be obtained.

The ingredient (B) of the present invention is one or more kinds selected from the group consisting of glycerin, 1,3-butylene glycol, 1,4-butylene glycol, propanediol, dipropylene glycol, diglycerin, threitol, erythritol, sorbitol and mannitol.

Among them, the ingredient (B) of the present invention is more preferably one or more kinds selected from the group consisting of glycerin, 1,3-butylene glycol, propanediol, dipropylene glycol, diglycerin and sorbitol, and further preferably one or more kinds selected from the group consisting of glycerin, 1,3-butylene glycol and dipropylene glycol.

The content of the ingredient (B) in the present invention is 0.1% or more by mass, preferably 0.5% or more by mass, and more preferably 1% or more by mass, and is 25% or less by mass, preferably 23% or less by mass, more preferably 20% or less by mass, and further preferably 15% or less by mass on the basis of the total amount of the skin external preparation. The specific range of the content of the ingredient (B) is preferably from 0.5 to 23% by mass, preferably from 1 to 20% by mass and more preferably from 1 to 15% by mass. Within this range, sufficient moisturizing feeling can be obtained, and moreover, excellent feeling on use without sticky feeling can obtained by the combination with the ingredient (A).

The total content of the ingredient (A) and the ingredient (B) in the present invention is preferably 0.2% or more by mass, more preferably 1% or more by mass and further preferably 2% or more by mass, and is preferably 35% or less by mass, more preferably 31% or less by mass, and further preferably 25% or less by mass, on the basis of the total amount of the skin external preparation. The specific range of the total content of the ingredient (A) and the ingredient (B) is preferably from 0.2 to 35% by mass, more preferably from 1 to 31% by mass and further preferably from 2 to 25% by mass. Within this range, the skin external preparation has excellent moisturizing effect.

The mass ratio of the content of the ingredient (A) relative to the total content of the ingredient (A) and the ingredient (B) [A/(A + B)] in the present invention is 0.03 or more, and preferably 0.05 or more, and is 0.8 or less, and preferably 0.5 or less. The specific range of A/(A + B) is preferably from 0.05 to 0.5. Within this range, the moisturizing effect of the ingredient (B) can be maintained, and excellent feeling on use without sticky feeling can be obtained.

A water-soluble polymer as the ingredient (C) is further formulated in the skin external preparation of the present invention. Formulating the ingredient (C) imparts viscosity to the skin external preparation, and the skin external preparation of the present invention has excellent feeling on use with elasticity and no stickiness.

The ingredient (C) used in the present invention is carboxyvinyl polymer and/or an acrylic acid-alkyl methacrylate copolymer.

The ingredient (C) may be used alone, or in combination of two or more.

The content of the ingredient (C) in the present invention is preferably is 0.01% or more by mass, more preferably 0.05% or more by mass and further preferably 0.1% or more by mass, and is preferably 1% or less by mass, more preferably 0.8% or less by mass and further preferably 0.5% or less by mass on the basis of the total amount of the skin external preparation. The specific range of the content of the ingredient (C) is from 0.01 to 1% by mass, more preferably from 0.05 to 0.8% by mass and further preferably from 0.1 to 0.5% by mass. Within this range, the skin external preparation of the present invention produces excellent feeling on use with elasticity and no sticky feeling.

In the skin external preparation of the present invention, optional ingredients that are ordinary used, may be suitably used in addition to the essential ingredients described above as long as the effects of the present invention are not impaired. Examples of such optional ingredients include, for example, various surfactants such as an anionic surfactant, an cationic surfactant and a nonionic surfactant, an oil, an ultraviolet absorber, a preservative, a moisturizing agent, a polymer, an amino acid derivative, a sugar derivative, a perfume, water, an alcohol, a thickener, a coloring material, a sequestering agent, an antioxidant, a drug, an extract derived from an animal or plant, and a pH adjusting agent.

The skin external preparation of the present invention can be manufactured in accordance with a conventional method. For example, it can be applied to any form such as skincare cosmetics such as a milky lotion, a cream, a toning lotion, a serum, a pack and a face wash; makeup cosmetics such as a lipstick, a foundation, a liquid foundation and a makeup pressed powder; hair cosmetics such as a hair shampoo, a hair rinse, a hair treatment, a conditioner, a hair dye and a hairdressing; cleansing cosmetics such as a face ash, a body shampoo and soap; and further a bath preparation. Of course, the skin external preparation is not limited thereto. Particularly, the skin external preparation of the present invention is preferably applied to a pharmaceutical product, a quasi-drug, a cosmetic, for example, as a skin external preparation such as a toning lotion, a milky lotion, a cream, a serum and a sunscreen.

[Examples]

Hereinafter, the present invention will be explained in detail with Examples.

Test Example 1 Moisturizing property test

A test for the moisturizing effect of using a combination of pentaerythritol, which is the ingredient (A), and one or more kinds selected from the group consisting of glycerin, 1,3-butylene glycol, propanediol, dipropylene glycol, 1,4-butylene glycol, diglycerin, threitol, erythritol, sorbitol and mannitol, which is the ingredient (B) of the present invention, was carried out.

(Method)

Test samples (Reference Example 1 and Comparative Examples 1 to 5) were prepared according to the formulations shown in Table 1.

The forearm of a subject was washed with a cleansing foam, and then acclimatized for 20 minutes in a room controlled to the temperature (25°C) and the humidity (45%). Then, the moisture amount in the stratum corneum of the forearm of the subject was measured using SKICON-200 (manufactured by IBS Japan Co., Ltd.).

Then, a test sample was applied in 20 $\mu$L/cm$^2$ respectively onto the forearm of the subject, and the test sample was dried by allowing to stand for 20 minutes. Then, 60 minutes after applying the test sample, the moisture amount in the stratum corneum was measured in the same manner as those described above.

The increased amount of the moisture in the stratum corneum was calculated relative to that before the start of application of the test sample, and the calculated amount was served to evaluation as the moisturizing effect.

[Table 1]

| (Content of Ingredient in Table is based on % by mass) | | | | | | |
|---|---|---|---|---|---|---|
| | Example 1* | Comparative Example 1 | Comparative Example 2 | Comparative Example 3 | Comparative Example 4 | Comparative Example 5 |
| A Pentaerythritol | 2 | 0 | 0 | 2 | 0 | 4 |
| B 1,3-Butylene glycol | 2 | 0 | 2 | 0 | 4 | 0 |
| 95% Ethanol | 10 | 10 | 10 | 10 | 10 | 10 |
| Pure water | 86 | 90 | 88 | 88 | 86 | 86 |
| Increased amount of moisture amount in stratum corneum 60 minutes after ($\mu$S) | 16.3 | -5.3 | 11.0 | -5.3 | 12.3 | -3.0 |
| *Reference | | | | | | |

The results of Table 1 show that the moisture amount in the stratum corneum in the control of Comparative Example 1 decreased 60 minutes after the application.

In the groups of Comparative Examples 2 and 4 containing the ingredient (B) only, increase of the moisture amount in the stratum corneum was recognized in comparison to the control. However, in the groups of Comparative Examples 3 and 5 containing the ingredient (A) only, the results were equal to the control. In contrast, in Reference Example 1 containing both the ingredient (A) and the ingredient (B), synergistic moisturizing effect was recognized, not additive effect shown in Comparative Examples 3 and 2. The result shown that addition of pentaerythritol to the ingredient (B), which has moisturizing property, can maintain or increase the moisturizing property despite decrease of the ingredient (B) in content.

Test Example 2 Half face comparative test in continuous use (softness of stratum corneum)

The half face comparative test in continuous use was carried out as described below using a serum containing pentaerythritol (ingredient (A) of the present invention), and the softness of the stratum corneum and the skin tone] in the buccal region were evaluated.

(Method for continuous use)

To healthy 11 male subjects 35 to 51 years old (44.0 years old on average), the pentaerythritol-containing serum (Example 2) was applied twice a day on a half face, and a placebo serum (without pentaerythritol; Comparative Example 6) was applied on the other half face by a double blind test. Evaluation was conducted 4 weeks and 8 weeks after continuous use, respectively. The formulations of the serums are shown in Table 2.

[Table 2]

| Ingredient (% by mass) | Example 2 | Comparative Example 6 |
| --- | --- | --- |
| Pentaerythritol | 4.0 | 0 |
| Dipropylene glycol | 10.0 | 10.0 |
| Glycerin | 5.0 | 5.0 |
| 95% Ethyl alcohol | 5.0 | 5.0 |
| Methyl polysiloxane | 2.0 | 2.0 |
| Polyoxyethylene hydrogenated castor oil | 0.5 | 0.5 |
| Phenoxyethanol | 0.3 | 0.3 |
| Carboxyvinyl polymer | 0.3 | 0.3 |
| Potassium hydroxide | 0.16 | 0.16 |
| Disodium edetate | 0.01 | 0.01 |
| Purified water | Balance | Balance |

(Evaluation method)

Softening effect of the stratum corneum was conducted using Venustron (manufactured by AXIOM) according to the method of Sakai, et al. (Sakai S, Sasai S et al., Characterization of the physical properties of the stratum corneum by a new tactile sensor., Skin Res Technol. 2000; 6(3): 128-134.). Venustron is an equipment that measures softness by bringing a probe vibrating at a certain frequency into contact with a test substance, and measures the amount of the frequency change ($\Delta f$(Hz)). $\Delta f$ increases (shift in plus direction) as the test substance is harder, and $\Delta f$ decreases as the test substance is softer (shift in minus direction).

Specifically, a measurement site was provided on the buccal region of a subject, and the same site was measured three times with Venustron (50 Hz of the probe frequency and maximum 3 mm of the indentation depth set up at the time of measurement). The frequency change when the load pressure of the probe was 2 g, was assumed as the softness $\Delta f$ (2 g) of the stratum corneum, and the average value thereof was calculated. Then, the softness of the stratum corneum before and after continuous use of the serum described above ($\Delta\Delta f$ (2 g)) was calculated from the formula below.

$$\Delta\Delta f\ (2\ g) = [\Delta f\ 4\ or\ 8\ weeks\ after\ continuous\ use]\ -$$

$$[\Delta f0\ before\ continuous\ use]$$

Herein, minus value of $\Delta\Delta fx$ (2 g) and high absolute value means strong effect of the softness of the stratum corneum.

In this test, the softness of the stratum corneum in the buccal region of a subject was measured using this method before continuous use and 4 weeks and 8 weeks after continuous use of the serums described above. The measurement results are represented by the average values of the subject.

[Table 3]

|  | $\Delta\Delta f$ (2 g) (Hz) | |
| --- | --- | --- |
|  | 4 weeks after | 8 weeks after |
| Example 2 | $-52.3 \pm 33.6$ | $-75.9 \pm 44.5$ * |
| Comparative Example 6 | $-44.7 \pm 39.8$ | $-44.1 \pm 49.2$ |
| * $p < 0.05$ vs Comparative Example 6 | | |

From the results of the softness of the stratum corneum in Table 3 described above, the softness of the stratum corneum in a group to which the pentaerythritol-containing serum of the present invention was applied (Example 2), was recognized to tend to increase 4 weeks after continuous use, and to increase significantly 8 weeks after continuous use in comparison to the placebo-applied group (Comparative Example 6).

Accordingly, it was found that the serum containing pentaerythritol of the present invention has excellent softening effect for the stratum corneum.

Test Example 3 Half face comparative test in continuous use (measurement of skin tone)

The half face comparative test in continuous use described below was carried out using the serum containing pentaerythritol, which is the ingredient (A) of the present invention, to evaluate the softness of the stratum corneum and the skin tone in the buccal region.

(Method for continuous use)

To healthy 22 female subjects ranging from 33 to 48 years old (38.1 years old on average), the pentaerythritol-containing serum (Example 3) was applied twice a day on a half face, and a placebo serum (without pentaerythritol; Comparative Example 6) was applied on the other half face by a double blind test. Evaluation was conducted 4 weeks and 8 weeks after continuous use respectively. The formulations of the serums are shown in Table 4.

[Table 4]

| Ingredient (% by mass) | Example 3 | Comparative Example 6 |
| --- | --- | --- |
| Pentaerythritol | 2.0 | 0 |
| Dipropylene glycol | 10.0 | 10.0 |
| Glycerin | 5.0 | 5.0 |
| 95% Ethyl alcohol | 5.0 | 5.0 |
| Methyl polysiloxane | 2.0 | 2.0 |
| Polyoxyethylene hydrogenated castor oil | 0.5 | 0.5 |
| Phenoxyethanol | 0.3 | 0.3 |
| Carboxyvinyl polymer | 0.3 | 0.3 |
| Potassium hydroxide | 0.16 | 0.16 |
| Disodium edetate | 0.01 | 0.01 |
| Purified water | Balance | Balance |

(Evaluation method)

The skin tone of the buccal region of the subject was measured using a spectrophotometer (Chroma Meter CM-2600d, manufactured by Konica Minolta, Inc.) for L value before continuous use and 4 weeks and 8 weeks after continuous use of the serum described above.

[Table 5]

|  | L value | | |
|---|---|---|---|
|  | Before continuous use | 4 weeks after | 8 weeks after |
| Example 3 | $62.4 \pm 1.7$ | $62.6 \pm 2.2$ ## | $62.9 \pm 2.3$ *, ## |
| Comparative Example 6 | $62.4 \pm 1.9$ | $62.0 \pm 2.4$ | $61.8 \pm 2.4$ * |
| *: $p < 0.05$ vs Before continuous use<br>##: $p < 0.01$ vs Comparative Example 6 | | | |

From the results of the skin tone measurement in Table 5 described above, it was recognized that L value of the group to which the pentaerythritol-containing serum of the present invention was applied (Example 3) increased significantly 4 weeks and 8 weeks after continuous use in comparison to the placebo-applied group (Comparative Example 6).

Accordingly, it was found that the serum of the present invention comprising pentaerythritol brightens the skin tone, and has excellent effect of improving the skin tone in comparison to Comparative Example 6 not formulated with pentaerythritol.

Meanwhile, there was no subject recognized to have skin stimulation reaction and skin sensitization reaction on the site applied with the serums of Examples 2 and 3, and Comparative Example 6 during the test period. It was confirmed that the product of the present invention is also safe in the form of the formulation.

Test Example 4 Test of feeling on use

A toning lotion was prepared according to the formulation shown in Table 6, and the feeling on use was evaluated by three expert judges. Three evaluation items of sticky feeling and moisturizing feeling were set up, and each of the evaluations was evaluated in accordance with the criteria described below, and the average points thereof are shown in Table 5 as well.

(Evaluation criteria)

(a) Absence of sticky feeling

5 Points: Very small sticky feeling
4 Points: Small sticky feeling
3 Points: Normal
2 Points: High sticky feeling
1 Point: Very high sticky feeling

(b) Moisturizing feeling

5 Points: Very high moist feeling of skin
4 Points: High moist feeling of skin
3 Points: Normal
2 Points: Small moist feeling of skin
1 Point: Very small moist feeling of skin

(c) Elasticity: Elasticity means resilient tight feeling when the skin is pressed with a finger.

5 Points: Very high elasticity of skin

7

4 Points: High elasticity of skin
3 Points: Normal
2 Points: Small elasticity of skin
1 Point: Very small elasticity of skin

[Table 6]

| (Content of Ingredient in Table is based on % by mass) | | | | | | | | | | | | | | | | | | |
|---|---|---|---|---|---|---|---|---|---|---|---|---|---|---|---|---|---|---|
| | Example | | | | | | | | | | | | | Comparative Example | | | | |
| | 4 | 5 | 6 | 7 | 8 | 9 | 10* | 11* | 12 | 13* | 14 | 15 | 16 | 7 | 8 | 9 | 10 | 11 |
| (A) Pentaerythritol | 2 | 2 | 2 | 0.5 | 1 | 4 | 0.1 | 5 | 2 | 2 | 2 | 2 | 2 | - | - | - | 2 | - |
| Raffinose | - | - | - | - | - | - | - | - | - | - | - | - | - | - | - | 2 | - | - |
| (B) Dipropylene glycol | 1 | 3 | 4 | 4.5 | 4 | 1 | 4.9 | 0.5 | 10 | 4 | - | - | - | - | 4 | 4 | - | 4 |
| (B) Glycerin | 1 | 3 | 4 | 5 | 5 | 1 | 5 | 0.5 | 5 | 4 | - | - | 4 | - | 4 | 4 | - | 4 |
| (B) 1,3-Butylene glycol | - | - | - | - | - | - | - | - | - | - | 4 | - | - | - | - | - | - | - |
| (B) Diglycerin | - | - | - | - | - | - | - | - | - | - | 4 | 4 | - | - | - | - | - | - |
| (B) Propanediol | - | - | - | - | - | - | - | - | - | - | - | 4 | - | - | - | - | - | - |
| (B) Sorbitol | - | - | - | - | - | - | - | - | - | - | - | - | 4 | - | - | - | - | - |
| (B) Mannitol | - | - | - | - | - | - | - | - | - | - | - | - | - | - | - | - | - | 2 |
| Polyoxyethylene hydrogenated castor oil (60E.O.) | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| (C) Carboxyvinyl polymer | 0.2 | 0.2 | 0.2 | 0.2 | 0.2 | 0.2 | 0.2 | 0.2 | 0.3 | - | 0.2 | 0.2 | 0.2 | 0.2 | 0.2 | 0.2 | 0.2 | 0.2 |
| Potassium hydroxide | 0.1 | 0.1 | 0.1 | 0.1 | 0.1 | 0.1 | 0.1 | 0.1 | 0.16 | - | 0.1 | 0.1 | 0.1 | 0.1 | 0.1 | 0.1 | 0.1 | 0.1 |
| Water | Balance | Balance | Balance | Balance | Balance | Balance | Balance | Balance | Balance | Balance | Balance | Balance | Balance | Balance | Balance | Balance | Balance | Balance |
| Evaluation item | | | | | | | | | | | | | | | | | | |
| (a) Absence of sticky feeling | 4.7 | 4.3 | 4.3 | 4.7 | 4.7 | 4.7 | 2.7 | 3.7 | 4.7 | 4.3 | 4.3 | 3.3 | 4.7 | 1.0 | 1.3 | 2.7 | 4.7 | 1.7 |

(continued)

| Evaluation item | | | | | | | | | | | | | | | | | | |
|---|---|---|---|---|---|---|---|---|---|---|---|---|---|---|---|---|---|---|
| (b) Moisturizing feeling | 3.7 | 3.3 | 4.3 | 3.7 | 4.0 | 3.3 | 3.7 | 1.3 | 4.3 | 3.0 | 4.7 | 3.7 | 3.7 | 1.0 | 4.3 | 4.3 | 1.3 | 3.7 |
| (c) Elasticity | 4.3 | 4.3 | 5.0 | 4.7 | 4.3 | 3.7 | 2.7 | 3.7 | 4.7 | 3.3 | 3.3 | 3.3 | 4.7 | 1.3 | 2.7 | 1.0 | 3.3 | 2.3 |
| A + B | 4 | 8 | 10 | 10 | 10 | 6 | 10 | 6 | 17 | 10 | 10 | 10 | 10 | 0 | 8 | 8 | 2 | 10 |
| A/(A + B) | 0.5 | 0.25 | 0.2 | 0.05 | 0.1 | 0.67 | 0.01 | 0.83 | 0.12 | 0.2 | 0.2 | 0.2 | 0.2 | - | - | - | - | - |
| *Reference | | | | | | | | | | | | | | | | | | |

From the results of Table 6, it was found that in Comparative Examples 8 and 11 not containing (A) pentaerythritol, the sticky feeling was not improved. In Comparative Example 9 containing raffinose instead of pentaerythritol, the sticky feeling was slightly improved but not sufficient, and the elasticity was not obtained. In Comparative Example 10 containing pentaerythritol only, the sticky feeling was fine, but sufficient moisturizing feeling or elasticity was not obtained. In contrast, in Examples containing (A) pentaerythritol and (B) one or more kinds selected from the group consisting of glycerin, 1,3-butylene glycol, propanediol, dipropylene glycol, 1,4-butylene glycol, diglycerin, threitol, erythritol, sorbitol and mannitol, and component (C) it was revealed that the sticky feeling was improved, so were the moisturizing feeling and the elasticity were also improved.

Hereinafter, there are formulation examples of the skin external preparation of the present invention. It is expected that excellent effect can be obtained in any formulation. The content of ingredient is based on % by mass.

Example 17 (Milky lotion) (Reference)

[Table 7]

| (Ingredient) | (Content) |
| --- | --- |
| (A) Pentaerythritol | 5.0 |
| (B) Dipropylene glycol | 10.0 |
| Bentonite | 0.5 |
| Glyceryl monostearate | 1.0 |
| Stearic acid | 0.5 |
| Behenyl alcohol | 0.3 |
| Cholesterol | 0.3 |
| Vaseline | 0.5 |
| Liquid paraffin | 10.0 |
| Methyl polysiloxane | 1.0 |
| (B) Conc. glycerin | 10.0 |
| Sodium N-stearoyl-L-glutamate | 0.2 |
| (C) Xanthan gum | 0.3 |
| Ascorbic acid 2-glucoside | 2.0 |
| Dipotassium glycyrrhizinate | 0.2 |
| Nicotinic acid amide | 1.0 |
| N-acetylglucosamine | 0.1 |
| N-methyl-L-serine | 0.1 |
| Phellodendron bark extract | 0.2 |
| Apricot kernel extract | 0.1 |
| Yeast extract | 0.1 |
| 1,2-Octanediol | 0.2 |
| Phenoxyethanol | 0.1 |
| Purified water | Balance |

Example 18 (Toning lotion) (Reference)

[Table 8]

| (Ingredient) | (Content) |
| --- | --- |
| (A) Pentaerythritol | 0.5 |
| (B) 1,3-Butylene glycol | 6.0 |
| (B) Glycerin | 10.0 |
| Ethanol | 8.0 |
| Polyoxyethylene hydrogenated castor oil (60E.O.) | 0.5 |
| Ascorbic acid | 1.0 |

(continued)

| (Ingredient) | (Content) |
| --- | --- |
| (B) Sorbitol | 1.0 |
| Citric acid | 0.1 |
| Sodium citrate | 0.3 |
| Purified water | Balance |

Example 19 (Cream) (Reference)

[Table 9]

| (Ingredient) | (Content) |
| --- | --- |
| (A) Pentaerythritol | 1.0 |
| (B) Dipropylene glycol | 10.0 |
| Stearic acid | 5.0 |
| Stearyl alcohol | 5.0 |
| Glyceryl tri-2-ethylhexanoate | 10.0 |
| Macadamia nut oil fatty acid cholesteryl | 4.0 |
| dl-$\alpha$-tocopherol acetate | 0.2 |
| Glyceryl monostearate | 3.0 |
| 1,2-Hexanediol | 1.0 |
| Potassium hydroxide | 0.2 |
| Phenoxyethanol | 0.3 |
| Methyl paraben | 0.1 |
| Perfume | q.s. |
| Purified water | Balance |

Example 20 (Cream)

[Table 10]

| (Ingredient) | (Content) |
| --- | --- |
| (A) Pentaerythritol | 2.0 |
| (B) Mannitol | 1.0 |
| (B) Conc. glycerin | 10.0 |
| Olive squalene | 10.0 |
| Palmitic acid | 2.0 |
| Hydrogenated palm kernel oil | 0.3 |
| Macadamia nut oil | 0.2 |
| Meadowfoam seed oil | 0.2 |
| Jojoba oil | 0.1 |
| Apricot kernel oil | 0.1 |
| Hydrogenated soybean phospholipid | 0.2 |
| Cetanol | 3.5 |
| Glyceryl monopalmitate | 2.0 |
| Phenoxyethanol | 0.1 |
| (C) Carboxyvinyl polymer | 0.2 |
| Potassium hydroxide | 0.2 |
| Purified water | Balance |

Example 21 (Sunscreen) (Reference)

[Table 11]

| (Ingredient) | (Content) |
|---|---|
| (A) Pentaerythritol | 3.0 |
| (B) 1,4-Butylene glycol | 1.0 |
| Methyl polysiloxane (1.5 mm$^2$/s) | 1.0 |
| Methyl polysiloxane (2.0 mm$^2$/s) | 2.0 |
| Methyl polysiloxane (6.0 mm$^2$/s) | 1.0 |
| Methyl phenylpolysiloxane | 2.0 |
| Methyl cyclopolysiloxane | 4.0 |
| Tris(trimethylsiloxy)methyl silane | 4.0 |
| Polyoxyethylene / methyl polysiloxane copolymer | 2.0 |
| Isododecane | 1.0 |
| Isohexadecane | 2.0 |
| Liquid isoparaffin | 2.0 |
| Isononyl isononanoate | 5.0 |
| Squalene | 1.0 |
| 2-Ethylhexyl p-methoxycinnamate | 5.0 |
| Dimethyldistearyl ammonium hectorite | 1.0 |
| Zinc oxide | 10.0 |
| Titanium oxide | 5.0 |
| Phenoxyethanol | 0.3 |
| Pure water | Balance |

Industrial Applicability

The present invention provides a skin external preparation, which generates no sticky feeling, has excellent stability and can be stored for a long time, and is excellent in feeling on use such as moisturizing feeling and elasticity, and further excellent in an effect of preventing and improving dullness of the skin.

**Claims**

1. A skin external preparation comprising the following ingredients (A) to (C):

    (A) from 0.1 to 10% by mass of pentaerythritol;
    (B) from 0.1 to 25% by mass of one or more kinds selected from the group consisting of glycerin, 1,3-butylene glycol, propanediol, dipropylene glycol, 1,4-butylene glycol, diglycerin, threitol, erythritol, sorbitol and mannitol; and
    (C) one or two or more of a water-soluble polymer selected from a carboxyvinyl polymer and an acrylic acid-alkyl methacrylate copolymer;

    wherein the mass ratio of the content of the ingredient (A) relative to the total content of the ingredient (A) and the ingredient (B) [A/(A + B)] is from 0.03 to 0.8.

2. The skin external preparation according to claim 1, wherein the ingredient (B) is one or more kinds selected from the group consisting of glycerin, 1,3-butylene glycol, propanediol, dipropylene glycol, diglycerin, and sorbitol.

3. The skin external preparation according to claim 1 or 2, wherein the total content of the ingredient (A) and the ingredient (B) is from 0.2 to 35% by mass on the basis of the total amount of the skin external preparation.

4. The skin external preparation according to any one of claims 1 to 3, wherein the mass ratio of the content of the ingredient (A) relative to the total content of the ingredient (A) and the ingredient (B) [A/(A + B)] is from 0.05 to 0.5.

5. Non-therapeutic use of a skin external preparation comprising the following ingredients (A) to (C) for improving elasticity of the skin or dull feeling of the skin:

(A) from 0.1 to 10% by mass of pentaerythritol;
(B) from 0.1 to 25% by mass of one or more kinds selected from the group consisting of glycerin, 1,3-butylene glycol, propanediol, dipropylene glycol, 1,4-butylene glycol, diglycerin, threitol, erythritol, sorbitol and mannitol; and
(C) one or two or more of a water-soluble polymer selected from a carboxyvinyl polymer and an acrylic acid-alkyl methacrylate copolymer;

wherein the mass ratio of the content of the ingredient (A) relative to the total content of the ingredient (A) and the ingredient (B) [A/(A + B)] is from 0.03 to 0.8 (the contents of ingredients (A) and (B) are on the basis of the total amount of the skin external preparation).

6. A nontherapeutic method for improving elasticity of the skin or dull feeling of the skin, the method comprising applying to the skin a composition comprising the following ingredients (A) to (C) :

(A) from 0.1 to 10% by mass of pentaerythritol;
(B) from 0.1 to 25% by mass of one or more kinds selected from the group consisting of glycerin, 1,3-butylene glycol, propanediol, dipropylene glycol, 1,4-butylene glycol, diglycerin, threitol, erythritol, sorbitol and mannitol; and
(C) one or two or more of a water-soluble polymer selected from a carboxyvinyl polymer and an acrylic acid-alkyl methacrylate copolymer;

wherein the mass ratio of the content of the ingredient (A) relative to the total content of the ingredient (A) and the ingredient (B) [A/(A + B)] is from 0.03 to 0.8.

**Patentansprüche**

1. Externes Hautpräparat, enthaltend die folgenden Bestandteile (A) bis (C):

(A) von 0,1 bis 10 Massen-% Pentaerythrit;
(B) von 0,1 bis 25 Massen-% von einer oder mehreren Arten, ausgewählt aus der Gruppe bestehend aus Glycerin, 1,3-Butylenglycol, Propandiol, Dipropylenglycol, 1,4-Butylenglycol, Diglycerin, Threit, Erythrit, Sorbit und Mannit; und
(C) ein oder zwei oder mehrere von einem wasserlöslichen Polymer, ausgewählt aus einem Carboxyvinylpolymer und einem Acrylsäure-Alkylmethacrylat-Copolymer,

worin das Massenverhältnis des Gehalts des Bestandteils (A) in Bezug auf den Gesamtgehalt des Bestandteils (A) und des Bestandteils (B), [A/(A + B)], von 0,03 bis 0,8 ist.

2. Externes Hautpräparat nach Anspruch 1, worin der Bestandteil (B) eine oder mehrere Arten ist, ausgewählt aus der Gruppe bestehend aus Glycerin, 1,3-Butylenglycol, Propandiol, Dipropylenglycol, Diglycerin, und Sorbit.

3. Externes Hautpräparat nach Anspruch 1 oder 2, worin der Gesamtgehalt des Bestandteils (A) und des Bestandteils (B) von 0,2 bis 35 Massen-% auf der Basis der Gesamtmenge des externen Hautpräparates ist.

4. Externes Hautpräparat nach einem der Ansprüche 1 bis 3, worin das Massenverhältnis des Gehaltes des Bestandteils (A) in Bezug auf den Gesamtgehalt des Bestandteils (A) und des Bestandteils (B), [A/(A + B)], von 0,05 bis 0,5 ist.

5. Nicht-therapeutische Verwendung eines externen Hautpräparates, enthaltend die folgenden Bestandteile (A) bis (C), zur Verbesserung der Elastizität der Haut oder eines stumpfen Gefühls der Haut:

(A) von 0,1 bis 10 Massen-% Pentaerythrit;
(B) von 0,1 bis 25 Massen-% von einer oder mehreren Arten, ausgewählt aus der Gruppe bestehend aus Glycerin, 1,3-Butylenglycol, Propandiol, Dipropylenglycol, 1,4-Butylenglycol, Diglycerin, Threit, Erythrit, Sorbit und Mannit; und

(C) ein oder zwei oder mehrere von einem wasserlöslichen Polymer, ausgewählt aus einem Carboxyvinylpolymer und einem Acrylsäure-Alkylmethacrylat-Copolymer,

worin das Massenverhältnis des Gehalts des Bestandteils (A) in Bezug auf den Gesamtgehalt des Bestandteils (A) und des Bestandteils (B), [A/(A + B)], von 0,03 bis 0,8 ist (die Gehalte der Bestandteile (A) und (B) sind auf der Basis der Gesamtmenge des externen Hautpräparates).

6. Nicht-therapeutisches Verfahren zur Verbesserung der Elastizität der Haut oder des stumpfen Gefühls der Haut, wobei das Verfahren das Auftragen einer Zusammensetzung mit den folgenden Bestandteilen (A) bis (C) auf die Haut enthält:

(A) von 0,1 bis 10 Massen-% Pentaerythrit;
(B) von 0,1 bis 25 Massen-% von einer oder mehreren Arten, ausgewählt aus der Gruppe bestehend aus Glycerin, 1,3-Butylenglycol, Propandiol, Dipropylenglycol, 1,4-Butylenglycol, Diglycerin, Threit, Erythrit, Sorbit und Mannit; und
(C) ein oder zwei oder mehrere von einem wasserlöslichen Polymer, ausgewählt aus einem Carboxyvinylpolymer und einem Acrylsäure-Alkylmethacrylat-Copolymer,

worin das Massenverhältnis des Gehalts des Bestandteils (A) in Bezug auf den Gesamtgehalt des Bestandteils (A) und des Bestandteils (B), [A/(A + B)], von 0,03 bis 0,8 ist.

## Revendications

1. Préparation externe pour la peau comprenant les composants (A) à (C) suivants :

(A) de 0,1 à 10 % en masse de pentaérythritol ;
(B) de 0,1 à 25 % en masse d'un ou plusieurs ingrédients choisis dans le groupe comprenant la glycérine, le 1,3-butylène glycol, le propanediol, le dipropylène glycol, le 1,4-butylène glycol, la diglycérine, le thréitol, l'érythritol, le sorbitol et le mannitol ; et
(C) un ou deux ou plus d'un polymère hydrosoluble choisi parmi un polymère carboxyvinylique et un copolymère d'acide acrylique et de méthacrylate d'alkyle ;

dans lequel le rapport massique de la teneur du composant (A) par rapport à la teneur totale du composant (A) et du composant (B) [A/(A+B)] est compris entre 0,03 et 0,8.

2. Préparation externe pour la peau selon la revendication 1, dans laquelle le composant (B) est un ou plusieurs ingrédients choisis dans le groupe comprenant la glycérine, le 1,3-butylène glycol, le propanediol, le dipropylène glycol, la diglycérine et le sorbitol.

3. Préparation externe pour la peau selon la revendication 1 ou 2, dans laquelle la teneur totale du composant (A) et du composant (B) est de 0,2 à 35 % en masse par rapport à la quantité totale de la préparation externe pour la peau.

4. Préparation externe pour la peau selon l'une quelconque des revendications 1 à 3, dans lequel le rapport massique de la teneur du composant (A) par rapport à la teneur totale du composant (A) et du composant (B) [A/(A+B)] est compris entre 0,05 et 0,5.

5. Utilisation non thérapeutique d'une préparation externe pour la peau comprenant les composants (A) à (C) suivants pour améliorer l'élasticité de la peau ou la sensation de teint terne de la peau :

(A) de 0,1 à 10 % en masse de pentaérythritol ;
(B) de 0,1 à 25 % en masse d'un ou plusieurs ingrédients choisis dans le groupe comprenant la glycérine, le 1,3-butylène glycol, le propanediol, le dipropylène glycol, le 1,4-butylène glycol, la diglycérine, le thréitol, l'érythritol, le sorbitol et le mannitol ; et
(C) un ou deux ou plus d'un polymère hydrosoluble choisi parmi un polymère carboxyvinylique et un copolymère d'acide acrylique et de méthacrylate d'alkyle ;

dans laquelle le rapport massique de la teneur du composant (A) par rapport à la teneur totale du composant (A) et du composant (B) [A/(A+B)] est compris entre 0,03 et 0,8 (les teneurs des composants (A) et (B) sont sur la base de la quantité totale de la préparation externe pour la peau).

6. Procédé non thérapeutique pour améliorer l'élasticité de la peau ou la sensation de teint terne de la peau, le procédé consistant à appliquer sur la peau une composition comprenant les composants (A) à (C) suivants :

(A) de 0,1 à 10 % en masse de pentaérythritol ;
(B) de 0,1 à 25 % en masse d'un ou plusieurs ingrédients choisis dans le groupe comprenant la glycérine, le 1,3-butylène glycol, le propanediol, le dipropylène glycol, le 1,4-butylène glycol, la diglycérine, le thréitol, l'érythritol, le sorbitol et le mannitol ; et
(C) un ou deux ou plus d'un polymère hydrosoluble choisi parmi un polymère carboxyvinylique et un copolymère d'acide acrylique et de méthacrylate d'alkyle ;

dans lequel le rapport massique de la teneur du composant (A) par rapport à la teneur totale du composant (A) et du composant (B) [A/(A+B)] est compris entre 0,03 et 0,8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4351749 B
- JP H10237105 A
- JP H1025234 A
- JP 4683861 B
- JP H0648914 B
- WO 2006061678 A

### Non-patent literature cited in the description

- **SAKAI S ; SASAI S et al.** Characterization of the physical properties of the stratum corneum by a new tactile sensor. *Skin Res Technol.,* 2000, vol. 6 (3), 128-134